(19) Europäisches Patentamt
European Patent Office
Office européen des brevets

(11) **EP 4 549 507 A1**

(12) **EUROPEAN PATENT APPLICATION**
published in accordance with Art. 153(4) EPC

(43) Date of publication:
07.05.2025 Bulletin 2025/19

(21) Application number: 23907477.6

(22) Date of filing: 28.11.2023

(51) International Patent Classification (IPC):
C08K 11/00 (2006.01)    C08K 5/17 (2006.01)
C08K 5/09 (2006.01)    C08K 5/053 (2006.01)
C08K 3/16 (2006.01)    C08F 20/06 (2006.01)
C08J 3/24 (2006.01)

(52) Cooperative Patent Classification (CPC):
C08F 20/06; C08J 3/24; C08K 3/16; C08K 5/053;
C08K 5/09; C08K 5/17; C08K 11/00

(86) International application number:
PCT/KR2023/019329

(87) International publication number:
WO 2024/136191 (27.06.2024 Gazette 2024/26)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 22.12.2022 KR 20220182257
23.11.2023 KR 20230164000

(71) Applicant: LG Chem, Ltd.
Yeongdeungpo-gu
Seoul 07336 (KR)

(72) Inventor: MIN, Ji Hong
Daejeon 34122 (KR)

(74) Representative: Plasseraud IP
104 Rue de Richelieu
CS92104
75080 Paris Cedex 02 (FR)

(54) **COMPOSITION OF SUPER ABSORBENT POLYMER AND PREPARATION METHOD THEREOF**

(57) The super absorbent polymer composition and the preparation method thereof according to the present disclosure are characterized by having excellent deodorizing ability while minimizing deterioration in physical properties of the super absorbent polymer by mixing persimmon extract, glycine, and a chelating agent into the super absorbent polymer after controlling thier combination and mixing ratio.

Processed by Luminess, 75001 PARIS (FR)

**Description**

**[TECHNICAL FIELD]**

CROSS-REFERENCE TO RELATED APPLICATION(S)

This application claims the benefits of Korean Patent Applications No. 10-2022-0182257 filed on December 22, 2022 and No. 10-2023-0164000 filed on November 23, 2023 with the Korean Intellectual Property Office, the disclosure of which is incorporated herein by reference in its entirety.

The present disclosure relates to a super absorbent polymer composition and a preparation method thereof. The present disclosure relates to a super absorbent polymer composition with deodorizing properties and a preparation method thereof.

**[BACKGROUND OF ART]**

A super absorbent polymer (SAP) is a type of synthetic polymeric material capable of absorbing 500 to 1000 times its own weight of moisture. Various manufacturers have denominated it with different names, such as SAM (Super Absorbency Material), AGM (Absorbent Gel Material), and the like. Such super absorbent polymers started to be practically applied in sanitary products, and they are now being widely used for water retaining soil products for gardening, water stop materials for the civil engineering and construction, sheets for raising seedling, fresh-keeping agents for food distribution fields, materials for poultices, or the like.

These super absorbent polymers have been widely used in the field of hygienic materials such as diapers or sanitary napkins. In such hygienic materials, the super absorbent polymer is generally contained in a state of being spread in the pulp. In recent years, however, continuous efforts have been made to provide hygienic materials such as diapers having a thinner thickness. As a part of such efforts, the development of so-called pulpless diapers and the like in which the pulp content is reduced or pulp is not used at all is being actively advanced.

In the case of the above-described hygienic materials in which the pulp content is reduced or pulp is not used, the super absorbent polymer is included in a relatively high ratio, so that the super absorbent polymer particles are inevitably included in multiple layers in the hygienic materials. In order for the entire super absorbent polymer particles included in multiple layers to absorb a liquid more efficiently such as urine, the super absorbent polymer basically needs to exhibit excellent absorption performance as well as a high absorption rate. Further, the absorbed liquid must not flow out even under external pressure, and permeability is also required to maintain its original shape even in a swollen state after absorbing liquid.

Accordingly, much research is being conducted to improve basic absorbency and water retention capacity of super absorbent polymers, such as performing surface cross-linking, etc.

However, super absorbent polymers can be used in hygienic materials, and in this case, there may be a problem of reduced usability due to the odor of absorbed liquids, such as human and pet excrement. In particular, there is a need to suppress both the odor contained in the absorbed liquid from the beginning and the odor caused by bacterial growth during the use of hygienic products containing the super absorbent polymer.

Accordingly, deodorizing substances were mixed with the super absorbent polymer previously. However, there was a problem in that absorbency was lowered by mixing an excessive amount of deodorizing substances in order to effectively suppress the odor, or the desired degree of deodorizing ability could not be achieved by focusing on antibacterial properties.

Accordingly, the demand for not only the basic properties of super absorbent polymers, which are absorbency and water retention capacity, but also odor suppression, is gradually increasing. Therefore, there is a need to provide a super absorbent polymer with excellent deodorizing ability.

**[DETAILED DESCRIPTION OF THE INVENTION]**

**[Technical Problem]**

In the present disclosure, there are provided a super absorbent polymer composition with deodorizing properties and a preparation method thereof.

More specifically, the present disclosure relates to a super absorbent polymer composition having excellent deodorizing ability while minimizing deterioration in physical properties of the super absorbent polymer by controlling the combination and mixing ratio of deodorizing substances, and a preparation method thereof.

**[Technical Solution]**

In order to solve the above problems, there is provided a super absorbent polymer composition including a super absorbent polymer comprising a base resin containing a cross-linked polymer in which an acrylic acid-based monomer having at least partially neutralized acidic groups and an internal cross-linking agent are cross-linking polymerized, and a surface cross-linked layer formed by further cross-linking the cross-linked polymer using a surface cross-linking agent on a surface of the base resin;

persimmon extract;
glycine; and
a chelating agent.

In addition, there is provided a preparation method of a super absorbent polymer composition including the steps of:

forming a hydrogel polymer by performing cross-linking polymerization of an acrylic acid-based monomer having at least partially neutralized acidic groups in the presence of an internal cross-linking agent and a polymerization initiator (step 1);
preparing a base resin containing a cross-linked polymer obtained by drying and pulverizing the hydrogel polymer (step 2);
preparing a mixture by mixing a surface cross-linking solution with the base resin (step 3); and
preparing a super absorbent polymer with a surface cross-linked layer formed on a surface of the base resin by heat-treating the mixture (step 4);
wherein a mixture containing persimmon extract and glycine is mixed with the surface cross-linking solution of step 3, or mixed with the super absorbent polymer on which the surface cross-linked layer is formed of step 4, and
the chelating agent is mixed with the hydrogel polymer of step 1, mixed with the surface cross-linking solution of step 3, or mixed with the super absorbent polymer on which the surface cross-linked layer is formed of step 4.

**[ADVANTAGEOUS EFFECTS]**

In the present disclosure, there are provided a super absorbent polymer composition having excellent deodorizing ability by applying a mixture containing persimmon extract and glycine in an optimal ratio and a chelating agent to the super absorbent polymer, and a preparation method of the same.

**[DETAILED DESCRIPTION OF THE EMBODIMENTS]**

The terminology used herein is for the purpose of describing particular embodiments only and is not intended to limit the invention. The singular forms are intended to include the plural forms as well, unless the context clearly indicates otherwise. It will be further understood that the terms "include", "have", or "possess" when used in this specification, specify the presence of stated features, steps, components, or combinations thereof, but do not preclude the presence or addition of one or more other features, steps, components, or combinations thereof.

As the present invention can be variously modified and have various forms, specific embodiments thereof are shown by way of examples and will be described in detail. However, it is not intended to limit the present invention to the particular form disclosed and it should be understood that the present invention includes all modifications, equivalents, and replacements within the idea and technical scope of the present invention.

As the present invention can be variously modified and have various forms, specific embodiments thereof are shown by way of examples and will be described in detail. However, it is not intended to limit the present invention to the particular form disclosed and it should be understood that the present invention includes all modifications, equivalents, and replacements within the idea and technical scope of the present invention.

Hereinafter, the super absorbent polymer composition and the preparation method of the same will be described in more detail according to specific embodiments of the invention.

The terminology used herein is only intended to refer to specific embodiments and is not intended to limit the present invention. Further, as used herein, the singular forms also include the plural forms unless the phrases clearly indicate the opposite.

For reference, the terminology "super absorbent polymer" is used to encompass all of the super absorbent polymer itself, and the polymer further processed, for example, surface cross-linking, fine reassembly, drying, pulverization, classification, etc., to be in a state suitable for commercialization, depending on the context.

In addition, "base resin" or "base resin powder" in this disclosure is made by drying and pulverizing a polymer in which acrylic acid-based monomers are polymerized into particles or powders, and refers to a polymer in which the surface modification or surface cross-linking to be described later has not been performed.

**(Super absorbent polymer composition)**

According to one embodiment of the present disclosure, there is provided a super absorbent polymer composition.

The super absorbent polymer composition includes a super absorbent polymer including a base resin containing a cross-linked polymer in which an acrylic acid-based monomer having at least partially neutralized acidic groups and an internal cross-linking agent are cross-linking polymerized, and a surface cross-linked layer formed by further cross-linking the cross-linked polymer using a surface cross-linking agent on a surface of the base resin; persimmon extract; glycine; and a chelating agent.

The acrylic acid-based monomer may be any monomer commonly used in the preparation of a super absorbent polymer. Specifically, the acrylic acid-based monomer may be a compound represented by the following Chemical Formula 1:

$$[\text{Chemical Formula 1}] \qquad R^1\text{-COOM}^1$$

in Chemical Formula 1,

$R^1$ is a C2 to C5 alkyl group having an unsaturated bond, and
$M^1$ is a hydrogen atom, a monovalent or divalent metal, an ammonium group, or an organic amine salt.

Preferably, the acrylic acid-based monomer may include at least one selected from the group consisting of acrylic acid, methacrylic acid, and a monovalent metal salt, a divalent metal salt, an ammonium salt, and an organic amine salt thereof.

The acrylic acid-based monomers may be those having acidic groups which are at least partially neutralized. Preferably, the acrylic acid-based monomer partially neutralized with an alkali substance such as sodium hydroxide, potassium hydroxide, ammonium hydroxide, or the like may be used.

Herein, a degree of neutralization of the monomer may be 40 to 95 mol%, 40 to 80 mol%, or 45 to 75 mol%. The range of the degree of neutralization can be adjusted according to final properties. An excessively high degree of neutralization causes the neutralized monomers to be precipitated, and thus polymerization may not readily occur. On the contrary, an excessively low degree of neutralization not only deteriorates absorbency of the polymer, but also gives the polymer hard-to-handle properties, such as those of an elastic rubber.

Meanwhile, it is performed in the presence of a cross-linking agent ("internal cross-linking agent") to improve physical properties of the polymer by polymerization of the acrylic acid-based monomer. The cross-linking agent is for internal cross-linking of the hydrogel polymer, and may be used separately from a "surface cross-linking agent" to be described later.

As the internal cross-linking agent, any compound can be used as long as it allows the introduction of cross-linking bonds during polymerization of the acrylic acid-based monomer. As a non-limiting example, the internal cross-linking agent may be a multifunctional cross-linking agent such as N,N'-methylenebisacrylamide, trimethylolpropane tri(meth)acrylate, ethyleneglycol di(meth)acrylate, polyethyleneglycol di(meth)acrylate, propyleneglycol di(meth)acrylate, polypropyleneglycol di(meth)acrylate, butanediol di(meth)acrylate, butyleneglycol di(meth)acrylate, diethyleneglycol di(meth)acrylate, hexanediol di(meth)acrylate, triethyleneglycol di(meth)acrylate, tripropyleneglycol di(meth)acrylate, tetraethyleneglycol di(meth)acrylate, dipentaerythritol pentaacrylate, glycerin tri(meth)acrylate, pentaerythritol tetraacrylate, triarylamine, allyl (meth)acrylate, ethylene glycol diglycidyl ether, propylene glycol, glycerin, or ethylene carbonate, and these may be used alone or in combination of two or more.

The internal cross-linking agent may be added at a concentration of 0.001 to 1 wt%, 0.01 to 0.8 wt%, or 0.1 to 0.7 wt% based on the monomer composition. That is, when the concentration of the internal cross-linking agent is too low, the absorption rate of the polymer may be lowered and gel strength may be weakened, which is not preferable. Conversely, when the concentration of the internal cross-linking agent is too high, the absorbency of the polymer is lowered, making it undesirable as an absorbent.

In addition, the base resin may further include additives such as a thickener, a plasticizer, a storage stabilizer, and an antioxidant, if necessary.

The surface cross-linking layer is formed by further cross-linking the cross-linked polymer using a surface cross-linking agent. As the surface cross-linking agent, any surface cross-linking agent capable of reacting with the functional group of the polymer, which has been conventionally used in the preparation of a super absorbent polymer, can be used without any particular limitation.

Preferably, in order to improve properties of the resulting super absorbent polymer, the surface cross-linking agent may be at least one selected from the group consisting of a polyalcohol-based compound; an epoxy compound; a polyamine compound; a haloepoxy compound; a condensation product of a haloepoxy compound; an oxazoline-based compound; a mono-, di- or polyoxazolidinone compound; a cyclic urea compound; a polyvalent metal salt; and an alkylene carbonate compound.

Specifically, as the polyalcohol-based compound, at least one selected from the group consisting of mono-, di-, tri-, tetra- or polyethylene glycol, monopropylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexanediol, and 1,2-cyclohexane dimethanol may be used.

In addition, as the epoxy compound, ethylene glycol diglycidyl ether, glycidol and the like may be used. As the polyamine compound, at least one selected from the group consisting of ethylenediamine, diethylenetriamine, triethylenetetraamine, tetraethylenepentamine, pentaethylenehexamine, polyethyleneimine and polyamide polyamine may be used.

Further, as the haloepoxy compound, epichlorohydrin, epibromohydrin, and $\alpha$-methylepichlorohydrin may be used. Meanwhile, as the mono-, di-, or polyoxazolidinone compound, 2-oxazolidinone may be used.

In addition, as the alkylene carbonate-based compound, ethylene carbonate, and the like may be used. These may be used alone or in combination with each other. Meanwhile, in order to increase the efficiency of the surface cross-linking process, one or more types of polyalcohol-based compounds having 2 to 10 carbon atoms can be used among these surface cross-linking agents.

The content of the added surface cross-linking agent may be appropriately selected depending on the type of surface cross-linking agent or reaction conditions, but is usually about 0.001 to about 5 parts by weight, preferably about 0.01 to about 3 parts by weight, more preferably about 0.05 to about 2 parts by weight based on 100 parts by weight of the polymer.

When the content of the surface cross-linking agent is too small, the surface cross-linking reaction hardly occurs. When it exceeds 5 parts by weight based on 100 parts by weight of the polymer, absorption capacity and physical properties may be deteriorated due to excessive surface cross-linking reaction.

Meanwhile, the surface cross-linking agent may additionally include an inorganic material. As the inorganic material, at least one inorganic material selected from the group consisting of silica, clay, alumina, silica-alumina composite, titania, zinc oxide and aluminum sulfate may be used. The inorganic material may be used in a powdery form or in a liquid form, and in particular, alumina powder, silica-alumina powder, titania powder, or nanosilica solution may be used. In addition, the inorganic material may be used in an amount of about 0.001 to about 1 parts by weight based on 100 parts by weight of the base resin.

The super absorbent polymer composition of one embodiment of the present disclosure may include persimmon extract and glycine as deodorizing substances. When the persimmon extract and glycine are applied to hygiene products, they have the effect of removing the odor inherent in the liquid absorbed by the hygiene products and removing additional odor that may occur due to bacterial growth during use.

The persimmon extract may be a substance extracted from persimmon tree leaves, persimmon tree stems, persimmon fruits, and persimmon tree roots. In addition, in the present disclosure, the "extract" refers to the result of extraction using an appropriate extraction solvent, and encompass all formulations that can be formed using the extracts, such as extracts obtained by extraction, diluted, or concentrated extracts, dried products obtained by drying extracts, purified products or fractions thereof, or mixtures thereof. However, the amount included, mixing amount, mixing ratio, etc. in the present disclosure are based on the solid content of the persimmon extract.

The glycine can capture odorous substances by chemically reacting with the odorous substances. Odorous substances generally have a small molecular weight, and when they react with glycine, they become substances with reduced or no odor. Thus, the glycine can effectively remove odor. In particular, the glycine is effective in reducing odor from aldehyde and ketone compounds.

The glycine may be included in an amount of 100 to 200 parts by weight with respect to 100 parts by weight of the persimmon extract based on the solid content. Preferably, the glycine may be included in an amount of 100 parts by weight or more, 120 parts by weight or more, 140 parts by weight or more, or 150 parts by weight or more to 200 parts by weight or less, 180 parts by weight or less, or 160 parts by weight with respect to 100 parts by weight of the persimmon extract based on the solid content.

The persimmon extract may be included in an amount of 0.005 to 0.050 parts by weight based on the solid content with respect to 100 parts by weight of the base resin. Specifically, the persimmon extract may be included in an amount of 0.005 parts by weight or more, 0.010 parts by weight or more, 0.015 parts by weight or more, 0.020 parts by weight or more, or 0.025 parts by weight or more to 0.050 parts by weight or less, 0.045 parts by weight or less, 0.040 parts by weight or less, 0.035 parts by weight or less, or 0.030 parts by weight or less based on the solid content with respect to 100 parts by weight of the base resin.

In addition, the glycine may be included in an amount of 0.005 to 0.050 parts by weight with respect to 100 parts by weight of the base resin. Specifically, the glycine may be included in an amount of 0.005 parts by weight or more, 0.010 parts by weight or more, 0.015 parts by weight or more, 0.020 parts by weight or more, 0.025 parts by weight or more, 0.030 parts by weight or more, or 0.035 parts by weight or more to 0.050 parts by weight or less, 0.045 parts by weight or less, or 0.040 parts by weight or less with respect to 100 parts by weight of the base resin.

In order to maintain the unique absorption properties of the super absorbent polymer, keep the color of the absorbent white from an aesthetic point of view, and exhibit deodorizing ability desired in the present disclosure, it is preferable that the persimmon extract or glycine, which are deodorizing substances, are included within the above content range.

When the solid content of the persimmon extract is more than 0.050 parts by weight, physical properties such as absorbency may be decreased and the color of the absorbent may change from white to yellow. When it is less than 0.005 parts by weight, deodorizing ability desired in the present disclosure may not be achieved.

The super absorbent polymer composition of one embodiment of the present disclosure may include a chelating agent. In the case of hygiene products containing a super absorbent polymer composition, additional odor may be generated due to the proliferation of bacteria when bacteria derived from the skin and the absorbed liquid meet, and the chelating agent can inhibit the growth of these bacteria.

The chelating agent may include an amino acetate-based chelating agent. Specifically, the amino acetate-based chelating agent may include at least one selected from the group consisting of ethylenediaminetetraacetic acid (EDTA), L-glutamic acid diacetic acid (GLDA), methyl glycine diacetic acid (MGDA), hydroxyethylethylenediaminetriacetic acid (HEDTA), ethanoldiglycine (EDG), diethylenetriaminepentaacetic acid (DTPA), and salts thereof. More specifically, the chelating agent may be ethylenediaminetetraacetic acid (EDTA) or L-glutamic acid diacetic acid (GLDA).

The chelating agent may be included in an amount of 0.1 to 2.0 parts by weight with respect to 100 parts by weight of the base resin. Specifically, the chelating agent may be included in an amount of 0.1 parts by weight or more, 0.3 parts by weight or more, 0.5 parts by weight or more, 0.7 parts by weight or more, or 1.0 parts by weight or more to 2.0 parts by weight or less, 1.5 parts by weight or less, or 1.3 parts by weight or less with respect to 100 parts by weight of the base resin.

In order to achieve antibacterial activity desired in the present disclosure while maintaining the unique absorption properties such as absorbency of the super absorbent polymer, and to exhibit deodorizing ability by inhibiting the growth of bacteria due to the antibacterial activity, it is preferable that the chelating agent is included within the above content range.

When the chelating agent is included in an amount of 0.1 parts by weight or less, it is difficult to achieve sufficient antibacterial activity to inhibit bacteria, and when the chelating agent is included in an amount of 2.0 parts by weight or more, physical properties of the super absorbent polymer itself may be deteriorated, such as a decrease in absorbency.

The super absorbent polymer composition may further include one or more additives selected from the group consisting of an organic acid, an iodine compound, glycerin, and zinc chloride.

The organic acid may be at least one selected from the group consisting of citric acid, acetic acid, formic acid, fumaric acid, lactic acid, and propionic acid. Specifically, the organic acid may be citric acid. The organic acid can exhibit deodorizing effects in super absorbent polymers, like the persimmon extract or glycine. Additionally, the organic acid has the effect of neutralizing ammonia dissolved in urine.

The iodine compound may be an iodized metal salt. The iodized metal salt may be added during the manufacturing process in the form of a solution in which one or more selected from the group consisting of Cul, Nal, and KI and $I_2$ are dissolved in water or in the form of a powder obtained by drying an aqueous solution. The iodized metal salt can also be added to super absorbent polymers to impart deodorizing properties, like persimmon extract or glycine. The iodized metal salt removes bad odors by oxidizing odorous substances, and acts on most odorous substances thereby generally effective.

The persimmon extract, glycine, and chelating agent may each independently be included inside the base resin or inside the surface cross-linked layer. Specifically, persimmon extract or glycine may be included inside the surface cross-linked layer, and the chelating agent may be included inside the base resin or inside the surface cross-linked layer.

The persimmon extract, glycine, chelating agent, organic acid, or iodine compound may be mixed with the super absorbent polymer in the form of an aqueous solution using water as a solvent, and may exist in a state physically incorporated into the super absorbent polymer. Alternatively, it may be mixed with the super absorbent polymer in the form of powder, and may exist in a state incorporated into the super absorbent polymer in the form of powder.

**(Preparation method of super absorbent polymer composition)**

According to one embodiment of the present disclosure, there is provided a preparation method of a super absorbent polymer composition.

The preparation method of a super absorbent polymer composition includes the steps of:

forming a hydrogel polymer by performing cross-linking polymerization of an acrylic acid-based monomer having at least partially neutralized acidic groups in the presence of an internal cross-linking agent and a polymerization initiator (step 1); preparing a base resin containing a cross-linked polymer obtained by pulverizing and drying the hydrogel polymer (step 2); preparing a mixture by mixing a surface cross-linking solution with the base resin (step 3); and preparing a super absorbent polymer with a surface cross-linked layer formed on a surface of the base resin by heat-treating the mixture (step 4);

wherein a mixture containing persimmon extract and glycine is mixed with the surface cross-linking solution of step 3, or mixed with the super absorbent polymer on which the surface cross-linked layer is formed of step 4, and the chelating agent is mixed with the hydrogel polymer of step 1, mixed with the surface cross-linking solution of step 3, or mixed with the super absorbent polymer on which the surface cross-linked layer is formed of step 4.

The preparation method of a super absorbent polymer includes a step of polymerizing an acrylic acid-based monomer to prepare a hydrogel polymer and a step of pulverizing the hydrogel polymer. Additionally, in order to improve various physical properties of the super absorbent polymer, a method of cross-linking the surface of the prepared super absorbent polymer is being used.

The present disclosure seeks to provide a super absorbent polymer composition having deodorizing properties by mixing the surface cross-linked super absorbent polymer with persimmon extract, glycine, and a chelating agent.

Hereinafter, the present invention will be described in detail for each step.

**(Step 1)**

The step 1 is a step of preparing a hydrogel polymer. Specifically, it is a step of forming a hydrogel polymer by performing cross-linking polymerization of a monomer composition containing an acrylic acid-based monomer having at least partially neutralized acidic groups.

The acrylic acid-based monomer may be any monomer commonly used in the preparation of a super absorbent polymer. Specifically, the acrylic acid-based monomer may be a compound represented by the following Chemical Formula 1:

$$[\text{Chemical Formula 1}] \qquad R^1\text{-COOM}^1$$

in Chemical Formula 1,

$R^1$ is a C2 to C5 alkyl group having an unsaturated bond, and
$M^1$ is a hydrogen atom, a monovalent or divalent metal, an ammonium group, or an organic amine salt.

Preferably, the acrylic acid-based monomer may include at least one selected from the group consisting of acrylic acid, methacrylic acid, and a monovalent metal salt, a divalent metal salt, an ammonium salt, and an organic amine salt thereof.

The acrylic acid-based monomers may be those having acidic groups which are at least partially neutralized. Preferably, the acrylic acid-based monomer partially neutralized with an alkali substance such as sodium hydroxide, potassium hydroxide, ammonium hydroxide, or the like may be used.

Herein, a degree of neutralization of the monomer may be 40 to 95 mol%, 40 to 80 mol%, or 45 to 75 mol%. The range of the degree of neutralization can be adjusted according to final properties. An excessively high degree of neutralization causes the neutralized monomers to be precipitated, and thus polymerization may not readily occur. On the contrary, an excessively low degree of neutralization not only deteriorates absorbency of the polymer, but also gives the polymer hard-to-handle properties, such as those of an elastic rubber.

The monomer composition may include a polymerization initiator commonly used in the preparation of a super absorbent polymer.

The polymerization initiator may be a thermal polymerization initiator or a photopolymerization initiator depending on the polymerization method. However, even in the case of performing the photopolymerization method, a certain amount of heat is generated by light irradiation such as ultraviolet irradiation. Further, a certain amount of heat may be generated with the progress of the polymerization reaction, which is an exothermic reaction, a thermal polymerization initiator can be further used.

For example, the photopolymerization initiator may be one or more compounds selected from the group consisting of benzoin ether, dialkyl acetophenone, hydroxyl alkylketone, phenyl glyoxylate, benzyl dimethyl ketal, acyl phosphine, and α-aminoketone. Meanwhile, as the specific example of acyl phosphine, commercial lucirin TPO, that is, 2,4,6-trimethyl-benzoyl-trimethyl phosphine oxide may be used. More various photopolymerization initiators are well disclosed in "UV Coatings: Basics, Recent Developments and New Application (Elsevier, 2007)" written by Reinhold Schwalm, p 115, and the present disclosure is not limited thereto.

In addition, one or more initiators selected from the group consisting of a persulfate-based initiator, an azo-based initiator, hydrogen peroxide, and ascorbic acid may be used as the thermal polymerization initiator. Specifically, sodium persulfate ($Na_2S_2O_8$), potassium persulfate ($K_2S_2O_8$), ammonium persulfate (($NH_4$)$_2S_2O_8$), and the like may be used as examples of the persulfate-based initiators; and 2,2-azobis(2-amidinopropane) dihydrochloride, 2,2-azobis-(N,N-dimethylene)isobutyramidine dihydrochloride, 2-(carbamoylazo)isobutylonitril, 2,2-azobis[2-(2-imidazolin-2-yl)propane] dihydrochloride, 4,4-azobis-(4-cyanovaleric acid), and the like may be used as examples of the azo-based initiators. More various thermal polymerization initiators are well disclosed in 'Principle of Polymerization (Wiley, 1981)' written by Odian, p 203, and the present disclosure is not limited thereto.

The polymerization initiator may be added at a concentration of 0.001 to 1 wt%, or 0.005 to 0.1 wt% based on the monomer composition. That is, when the concentration of the polymerization initiator is too low, the polymerization rate may become slow and thus a large amount of residual monomer may be extracted from the final product, which is not preferable. On the contrary, when the concentration of the polymerization initiator is too high, a polymer chain forming a network may become short, and thus, the physical properties of polymer may be degraded, such as increase in the content of water-soluble components and decrease in absorbency under pressure, which is not preferable.

Meanwhile, the polymerization of the monomer composition is performed in the presence of a cross-linking agent ("internal cross-linking agent") to improve physical properties of the polymer by polymerization of the acrylic acid-based monomer. The cross-linking agent is for internal cross-linking of the hydrogel polymer, and may be used separately from a "surface cross-linking agent" to be described later.

As the internal cross-linking agent, any compound can be used as long as it allows the introduction of cross-linking bonds during polymerization of the acrylic acid-based monomer. As a non-limiting example, the internal cross-linking agent may be a multifunctional cross-linking agent such as N,N'-methylenebisacrylamide, trimethylolpropane tri(meth)acrylate, ethyleneglycol di(meth)acrylate, polyethyleneglycol di(meth)acrylate, propyleneglycol di(meth)acrylate, polypropyleneglycol di(meth)acrylate, butanediol di(meth)acrylate, butyleneglycol di(meth)acrylate, diethyleneglycol di(meth)acrylate, hexanediol di(meth)acrylate, triethyleneglycol di(meth)acrylate, tripropyleneglycol di(meth)acrylate, tetraethyleneglycol di(meth)acrylate, dipentaerythritol pentaacrylate, glycerin tri(meth)acrylate, pentaerythritol tetraacrylate, triarylamine, allyl (meth)acrylate, ethylene glycol diglycidyl ether, propylene glycol, glycerin, or ethylene carbonate, and these may be used alone or in combination of two or more.

The internal cross-linking agent may be added at a concentration of 0.001 to 1 wt%, 0.01 to 0.8 wt%, or 0.1 to 0.7 wt% based on the monomer composition. That is, when the concentration of the internal cross-linking agent is too low, the absorption rate of the polymer may be lowered and gel strength may be weakened, which is not preferable. Conversely, when the concentration of the internal cross-linking agent is too high, the absorbency of the polymer is lowered, making it undesirable as an absorbent.

In addition, the cross-linking polymerization of the monomer composition may be performed in the presence of a foaming agent depending on the need and degree of improvement in absorption rate. Such a foaming agent may be decomposed during the cross-linking polymerization to generate gas, thereby forming pores in the hydrogel polymer. As a result, when such a foaming agent is additionally used, a more developed porous structure is formed in the super absorbent polymer, so that the absorption rate of the super absorbent polymer can be further improved.

As a non-limiting example, the foaming agent may include at least one compound selected from the group consisting of sodium bicarbonate, sodium carbonate, potassium bicarbonate, potassium carbonate, calcium bicarbonate, calcium carbonate, magnesium bicarbonate, magnesium carbonate, azodicarbonamide (ADCA), dinitroso pentamethylene tetramine (DPT), p,p'-oxybis(benzenesulfonyl hydrazide) (OBSH), p-toluenesulfonyl hydrazide (TSH), sucrose stearate, sucrose palmitate, and sucrose laurate.

The foaming agent may be present in the monomer composition in an amount of 1000 to 4000 ppmw. More specifically, it may be present in an amount of 1000 ppm or more, 1100 ppmw or more, or 1200 ppmw or more; and 4000 ppmw or less, 3500 ppmw or less, or 3000 ppmw or less.

In addition, the monomer composition may further include a thickener, a plasticizer, a preservation stabilizer, an antioxidant, or the like, if necessary.

In addition, such a monomer composition may be prepared in the form of a solution in which raw materials such as the above-described acrylic acid-based monomer, polymerization initiator, internal cross-linking agent, and foaming agent are dissolved in a solvent.

At this time, any solvent which can dissolve the raw materials may be used without limitation, and for example, water, ethanol, ethyleneglycol, diethyleneglycol, triethyleneglycol, 1,4-butanediol, propyleneglycol, ethyleneglycol monobutylether, propyleneglycol monomethylether, propyleneglycol monomethylether acetate, methylethylketone, acetone, methylamylketone, cyclohexanone, cyclopentanone, diethyleneglycol monomethylether, diethyleneglycol ethylether, toluene, xylene, butyrolactone, carbitol, methylcellosolve acetate, N,N-dimethylacetamide, or a mixture thereof can be used.

The formation of the hydrogel polymer by polymerizing the monomer composition may be performed by a conventional polymerization method, and the process is not particularly limited.

As a non-limiting example, the polymerization method is largely divided into the thermal polymerization and the photopolymerization according to an energy source of the polymerization. In the case of thermal polymerization, it is generally conducted in a reactor equipped with an agitation spindle, such as a kneader. In the case of photopolymerization, it may be conducted in a reactor equipped with a movable conveyor belt.

For example, the hydrogel polymer may be obtained by introducing the monomer composition into a reactor equipped with an agitation spindle such as a kneader, followed by supplying hot air, or heating the reactor to perform thermal polymerization. The hydrogel polymer may be discharged to a reactor outlet in the form of particles with several centimeters to several millimeters depending on a shape of the agitation spindle provided in the reactor. Specifically, a shape of the hydrogel polymer obtained may vary depending on the concentration and injection rate of the monomer mixture to be injected, and a hydrogel polymer having a (weight average) particle diameter of 2 to 50 mm may be usually obtained.

As another example, when photopolymerization is performed on the monomer composition in the reactor equipped with a movable conveyor belt, a hydrogel polymer in the form of a sheet may be obtained. At this time, a thickness of the sheet may vary depending on the concentration and injection rate of the monomer composition to be injected. It is preferable to adjust the thickness to 0.5 to 10 cm in order to ensure the production rate while allowing the entire sheet to be polymerized evenly.

The hydrogel polymer formed in this way may exhibit a moisture content of 40 to 80 wt%. At this time, the moisture content is the weight occupied by moisture in the total weight of the hydrogel polymer, and it means a value of which the weight of the dried polymer is subtracted from the weight of the hydrogel polymer. Specifically, the moisture content is defined as a value calculated by measuring the weight loss due to moisture evaporation from the polymer in the process of increasing the temperature of the polymer for drying through infrared heating. At this time, the drying condition is as follows: the temperature is increased to about 180 °C and maintained at 180 °C, and the total drying time is 20 minutes including 5 minutes of a heating step.

A chelating agent may be mixed with the hydrogel polymer prepared in step 1 before the drying, pulverization, and classification in step 2. In step 1, the hydrogel polymer can be prepared in the form of a sheet, and mixed by chopping the sheet while spraying a chelating agent.

The chelating agent may include an amino acetate-based chelating agent. Specifically, the amino acetate-based chelating agent may include at least one selected from the group consisting of ethylenediaminetetraacetic acid (EDTA), L-glutamic acid diacetic acid (GLDA), methyl glycine diacetic acid (MGDA), hydroxyethylethylenediaminetriacetic acid (HEDTA), ethanoldiglycine (EDG), diethylenetriaminepentaacetic acid (DTPA), and salts thereof. More specifically, the chelating agent may be ethylenediaminetetraacetic acid (EDTA) or L-glutamic acid diacetic acid (GLDA).

The chelating agent may be included in an amount of 0.1 to 2.0 parts by weight with respect to 100 parts by weight of the base resin. Specifically, the chelating agent may be used in an amount of 0.1 parts by weight or more, 0.3 parts by weight or more, 0.5 parts by weight or more, 0.7 parts by weight or more, or 1.0 parts by weight or more to 2.0 parts by weight or less, 1.5 parts by weight or less, or 1.3 parts by weight or less with respect to 100 parts by weight of the base resin.

In order to exhibit deodorizing ability by inhibiting the growth of bacteria desired in the present disclosure while maintaining the unique absorption properties of the super absorbent polymer, it is preferable that the chelating agent is included within the above content range.

The chelating agent may be mixed in the base resin in an aqueous solution or in powder form.

**(Step 2)**

The step 2 of the present disclosure is a step of drying, pulverizing, and classifying the hydrogel polymer prepared in step 1 to form a base resin powder.

Specifically, the present disclosure may further include a step of coarsely pulverizing the hydrogel polymer before drying in order to improve the absorption rate of the super absorbent polymer. The coarse pulverization not only increases the drying efficiency of the hydrogel polymer, but also affects morphology of the super absorbent polymer, which can affect various physical properties of the super absorbent polymer including the absorption rate. Hereinafter, in order to distinguish it from pulverization after drying, the term 'coarse pulverization' is used for pulverization before drying.

At this time, there is no limitation in the configuration of the pulverizing machine used in the pulverization step. Specifically, at least one pulverizing machine selected from the group consisting of a vertical pulverizer, a turbo cutter, a turbo grinder, a rotary cutter mill, a cutter mill, a disc mill, a shred crusher, a crusher, a chopper, and a disc cutter may be used, but the present disclosure is not limited thereto.

Herein, the hydrogel polymer may be coarsely pulverized so that the particle diameter is about 2 mm to about 10 mm. Pulverization to a particle diameter of less than 2 mm is not technically easy due to the high moisture content of the hydrogel polymer, and a phenomenon of agglomeration between the pulverized particles may occur. On the other hand, when pulverized to a particle diameter of more than 10 mm, an increase in efficiency of the subsequent drying step may be insignificant.

The drying may be performed at a temperature of 120 to 250 °C, 140 to 200 °C, or 150 to 190 °C. In this case, the drying temperature may be defined as the temperature of the heating medium supplied for drying or the temperature inside the drying reactor including the heating medium and the polymer in the drying process. When the drying time is prolonged due to the low drying temperature, the process efficiency is lowered. In order to prevent this, the drying temperature is preferably 120 °C or higher. In addition, when the drying temperature is excessively high, the surface of the hydrogel polymer is excessively dried, which may increase the generation of fines in the subsequent pulverization step, thereby decreasing physical properties of the final polymer. In order to prevent this, the drying temperature is preferably 250 °C or lower.

Herein, the drying time may be about 20 minutes to about 90 minutes in consideration of process efficiency and physical properties of the polymer, but is not limited thereto.

The drying can be performed using a common medium, for example, by the method of hot air provision, infrared radiation, microwave radiation, UV ray radiation, and the like.

In addition, the drying is preferably performed so that the dried polymer has a moisture content of 0.1 to 10 wt%. That is, when the moisture content of the dried polymer is less than 0.1 wt%, excessive drying may increase manufacturing costs and cause degradation of the cross-linked polymer, which is not desirable. Additionally, when the moisture content of the dried polymer exceeds 10 wt%, defects may occur in subsequent processes, which is not desirable.

Subsequently, the dried hydrogel polymer can be pulverized. This is a step to optimize the surface area of the base resin powder and super absorbent polymer. The pulverization may be performed so that the particle diameter of the pulverized polymer is 150 to 850 $\mu$m.

As the pulverizing machine, a pin mill, a hammer mill, a screw mill, a roll mill, a disc mill, a jog mill, or the like may be used.

In order to control physical properties of the super absorbent polymer to be prepared as a final product, a step of selectively classifying particles having a particle diameter of 150 to 850 $\mu$m from the polymer particles obtained through the pulverization step is performed.

After the above classification step, a base resin powder can be obtained. This base resin powder may have a particle diameter of 150 to 850 $\mu$m, and may contain 2 wt% or less, or 1 wt% or less of fine powder with a particle diameter of less than 150 $\mu$m.

In addition, after drying and pulverizing to obtain a base resin powder, a step of mixing zirconium phosphate may be further included. At this time, zirconium phosphate can be dry-mixed. That is, zirconium phosphate can be physically mixed as a solid content in a dry manner in the base resin before surface cross-linking. Zirconium phosphate is a substance that can physically adsorb odorous substances. Accordingly, additional deodorizing effect can be achieved by further mixing zirconium phosphate with the base resin powder.

At this time, zirconium phosphate may be included in an amount of 5.0 parts by weight or less with respect to 100 parts by weight of the base resin. Zirconium phosphate is additionally used for increasing deodorizing ability and may not be included. When included, it may be included in an amount of 0.5 parts by weight or more, 0.8 parts by weight or more, 1.0 parts by weight or more, 1.3 parts by weight or more, 1.5 parts by weight or more, or 1.8 parts by weight or more and 5.0 parts by weight or less, 4.0 parts by weight or less, 3.0 parts by weight or less, or 2.0 parts by weight or less. When too little zirconium phosphate is mixed, there may be a problem in that the deodorizing effect by the zirconium phosphate is insignificant, and when too much zirconium phosphate is included, there may be a problem in that absorbency under pressure is reduced due to poor surface cross-linking.

**(Step 3)**

The step 3 of the present disclosure is a step of mixing a surface cross-linking solution with the base resin powder prepared in step 2.

The surface cross-linking solution used in step 3 contains a surface cross-linking agent, and the surface cross-linking agent is not particularly limited as long as it is a compound capable of reacting with the functional group of the polymer, which is generally used for surface cross-linking of super absorbent polymers.

Preferably, in order to improve properties of the resulting super absorbent polymer, the surface cross-linking agent may be at least one selected from the group consisting of a polyalcohol-based compound; an epoxy compound; a polyamine compound; a haloepoxy compound; a condensation product of a haloepoxy compound; an oxazoline-based compound; a mono-, di- or polyoxazolidinone compound; a cyclic urea compound; a polyvalent metal salt; and an alkylene carbonate compound.

Specifically, as the polyalcohol-based compound, at least one selected from the group consisting of mono-, di-, tri-, tetra- or polyethylene glycol, monopropylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexanediol, and 1,2-cyclohexane dimethanol may be used.

In addition, as the epoxy compound, ethylene glycol diglycidyl ether and glycidol may be used. As the polyamine compound, at least one selected from the group consisting of ethylenediamine, diethylenetriamine, triethylenetetraamine, tetraethylenepentamine, pentaethylenehexamine, polyethyleneimine and polyamide polyamine may be used.

Further, as the haloepoxy compound, epichlorohydrin, epibromohydrin, and $\alpha$-methylepichlorohydrin may be used. Meanwhile, as the mono-, di-, or polyoxazolidinone compound, 2-oxazolidinone may be used.

In addition, as the alkylene carbonate-based compound, ethylene carbonate may be used. These may be used alone or in combination with each other. Meanwhile, in order to increase the efficiency of the surface cross-linking process, one or more types of polyalcohol-based compounds having 2 to 10 carbon atoms can be used among these surface cross-linking agents.

The content of the added surface cross-linking agent may be appropriately selected depending on the type of surface cross-linking agent or reaction conditions, but is usually about 0.001 to about 5 parts by weight, preferably about 0.01 to about 3 parts by weight, more preferably about 0.05 to about 2 parts by weight based on 100 parts by weight of the polymer.

When the content of the surface cross-linking agent is too small, the surface cross-linking reaction hardly occurs. When it exceeds 5 parts by weight based on 100 parts by weight of the polymer, absorption capacity and physical properties may be deteriorated due to excessive surface cross-linking reaction.

Meanwhile, a step of forming a surface cross-linked layer may be performed by additionally including an inorganic material in the surface cross-linking solution. As the inorganic material, at least one inorganic material selected from the group consisting of silica, clay, alumina, silica-alumina composite, titania, zinc oxide and aluminum sulfate may be used. The inorganic material may be used in a powdery form or in a liquid form, and in particular, alumina powder, silica-alumina powder, titania powder, or nanosilica solution may be used. In addition, the inorganic material may be used in an amount of about 0.001 to about 1 parts by weight based on 100 parts by weight of the base resin.

Additionally, a mixture containing persimmon extract and glycine may be mixed in the base resin together with the surface cross-linking solution. The persimmon extract and glycine can chemically combine with volatile odorous substances such as aldehydes, thereby imparting deodorizing ability to the super absorbent polymer.

The glycine may be included in an amount of 100 to 200 parts by weight with respect to 100 parts by weight of the persimmon extract based on the solid content. Preferably, the glycine may be included in an amount of 100 parts by weight or more, 120 parts by weight or more, 140 parts by weight or more, or 150 parts by weight or more to 200 parts by weight or less, 180 parts by weight or less, or 160 parts by weight with respect to 100 parts by weight of the persimmon extract based on the solid content.

The persimmon extract may be included in an amount of 0.005 to 0.050 parts by weight based on the solid content with respect to 100 parts by weight of the base resin. Specifically, the persimmon extract may be included in an amount of 0.005 parts by weight or more, 0.010 parts by weight or more, 0.015 parts by weight or more, 0.020 parts by weight or more, or 0.025 parts by weight or more to 0.050 parts by weight or less, 0.045 parts by weight or less, 0.040 parts by weight or less, 0.035 parts by weight or less, or 0.030 parts by weight or less based on the solid content with respect to 100 parts by weight of the base resin.

In addition, the glycine may be included in an amount of 0.005 to 0.050 parts by weight with respect to 100 parts by weight of the base resin. Specifically, the glycine may be included in an amount of 0.005 parts by weight or more, 0.010 parts by weight or more, 0.015 parts by weight or more, 0.020 parts by weight or more, 0.025 parts by weight or more, 0.030 parts by weight or more, or 0.035 parts by weight or more to 0.050 parts by weight or less, 0.045 parts by weight or less, or 0.040 parts by weight or less with respect to 100 parts by weight of the base resin.

In order to exhibit deodorizing ability desired in the present disclosure while maintaining the unique absorption properties of the super absorbent polymer, it is preferable that the persimmon extract or glycine, which are deodorizing substances, are included within the above content range.

The deodorizing substance containing persimmon extract and glycine may be mixed in the base resin in an aqueous solution or in powder form.

In addition, a chelating agent may be mixed in the base resin along with the surface cross-linking solution. For details on the type of chelating agent, mixing amount, and mixing form, refer to the step 1.

Meanwhile, the method of mixing the surface cross-linking solution with the base resin is not particularly limited, and can be appropriately selected as long as it is a method that can evenly mix it with the base resin.

For example, a method of adding the surface cross-linking solution and the base resin in a reactor for mixing, a method of spraying the surface cross-linking solution onto the base resin, or a method of mixing the base resin and the surface cross-linking solution while continuously providing them to a continuously operating mixer may be used.

At this time, the surface cross-linking solution may be an aqueous solution, and the solid content in the solution may be 1 wt% or more, 3 wt% or more, 5 wt% or more, or 10 wt% or more, and 50 wt% or less, 30 wt% or less, or 20 wt% or less for even dispersion in the base resin and prevention of agglomeration of the base resin.

**(Step 4)**

The step 4 is a step to further improve physical properties of the super absorbent polymer by reacting the base resin and the surface cross-linking solution to form an interpenetrating polymer network on the surface of the cross-linked polymer contained in the base resin. Through this surface modification, a surface cross-linked layer is formed on the surface of the pulverized base resin particles.

The formation of the surface cross-linked layer can be performed by a conventional method of increasing the cross-linking density of the surface of the polymer particles, for example, by a method of mixing a surface cross-linking solution containing a surface cross-linking agent with the pulverized polymer, followed by heat treatment to perform a cross-linking reaction.

The step 4 may be performed at a temperature of about 80 °C to about 250 °C. More specifically, the surface cross-linking process may be performed at a temperature of about 100 °C to about 220 °C, about 110 °C to about 200 °C, or about 120 °C to about 190 °C for about 10 minutes to about 2 hours, or for about 20 minutes to about 60 minutes. When the cross-linking reaction temperature is less than 160 °C or the reaction time is too short, the surface cross-linking reaction may not occur properly and permeability may be lowered. When it exceeds 200 °C or the reaction time is too long, water retention capacity may be reduced.

The heating means for the surface cross-linking reaction is not particularly limited. It is possible to provide a thermal media thereto or provide a heat source directly thereto. At this time, usable thermal media may be a heated fluid such as steam, hot air, hot oil, and the like, but the present invention is not limited thereto. Furthermore, the temperature of the thermal media provided thereto may be properly selected in consideration of the means of the thermal media, heating speed, and target temperature of heating. Meanwhile, an electric heater or a gas heater may be used as the heat source provided directly, but the present disclosure is not limited thereto.

Additionally, a mixture containing persimmon extract and glycine may be mixed with the super absorbent polymer on which the surface cross-linked layer is formed. For details on the mixing amount, and mixing form of persimmon extract and glycine, refer to the step 3.

In addition, a chelating agent may be mixed with the super absorbent polymer on which the surface cross-linked layer is formed. For details on the type of chelating agent, mixing amount, and mixing form, refer to the step 1.

In addition, one or more additives selected from the group consisting of an organic acid, an iodine compound, glycerin, and zinc chloride may be further mixed with the super absorbent polymer on which the surface cross-linked layer is formed.

The organic acid may be at least one selected from the group consisting of citric acid, acetic acid, formic acid, fumaric acid, lactic acid, and propionic acid. Specifically, the organic acid may be citric acid. The organic acid can exhibit deodorizing effects in super absorbent polymers, like the persimmon extract or glycine.

The iodine compound may be an iodized metal salt. The iodized metal salt may be added during the manufacturing process in the form of a solution in which one or more selected from the group consisting of CuI, NaI, and KI and $I_2$ are dissolved in water or in the form of a powder obtained by drying an aqueous solution. The iodized metal salt can also be added to super absorbent polymers to impart deodorizing properties, like the deodorizing substances. The iodized metal salt removes bad odors by oxidizing odorous substances, and acts on most odorous substances thereby generally effective.

The organic acid or iodine compound may be mixed in an aqueous solution using water as a solvent or in powder form.

When deodorizing substances and a chelating agent are added later to the super absorbent polymer on which the surface cross-linked layer is formed as an aqueous solution, a drying step may be additionally performed.

Hereinafter, preferred embodiments are presented to facilitate the understanding of the invention. However, the following examples are for illustrative purposes only and are not intended to limit the present disclosure.

**Preparation Example 1** - **Mixture A**

Persimmon extract and glycine were prepared as an aqueous solution. At this time, 5 parts by weight of persimmon extract based on the solid content and 5 parts by weight of glycine were mixed with respect to 100 parts by weight of the aqueous solution.

**Preparation Example 2** - **Mixture B**

Persimmon extract and glycine were prepared as an aqueous solution. At this time, 5 parts by weight of persimmon extract based on the solid content and 7 parts by weight of glycine were mixed with respect to 100 parts by weight of the aqueous solution.

**Comparative Preparation Example 1** - **Mixture C**

Persimmon extract and methionine were prepared as an aqueous solution. At this time, 5 parts by weight of persimmon extract based on the solid content and 5 parts by weight of methionine were mixed with respect to 100 parts by weight of the aqueous solution.

**Comparative Example 1**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

**Comparative Example 2**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

**Comparative Example 3**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.3 parts by weight of a mixture A in an aqueous solution was mixed with the surface-treated super absorbent polymer with respect to 100 parts by weight of the base resin. Afterwards, a drying step was performed at 80 °C for 25 minutes.

**Comparative Example 4**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.3 parts by weight of a mixture C in an aqueous solution was mixed with the surface-treated super absorbent polymer with respect to 100 parts by weight of the base resin. Afterwards, a drying step was performed at 80 °C for 25 minutes.

**Comparative Example 5**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

An aqueous glycine solution was mixed with the surface-treated super absorbent polymer so that 0.015 parts by weight of glycine was added with respect to 100 parts by weight of the base resin. Afterwards, a drying step was performed at 80 °C for 25 minutes.

**Comparative Example 6**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

An aqueous solution of persimmon extract was mixed with the surface-treated super absorbent polymer so that the solid content of persimmon extract is 0.015 parts by weight with respect to 100 parts by weight of the base resin. Afterwards, a drying step was performed at 80 °C for 25 minutes.

**Comparative Example 7**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

An aqueous glycine solution was mixed with the surface-treated super absorbent polymer so that 0.015 parts by weight of glycine was added with respect to 100 parts by weight of the base resin. Afterwards, a drying step was performed at 80 °C for 25 minutes.

**Example 1**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.1 parts by weight of a mixture A in an aqueous solution was mixed with the surface-treated super absorbent polymer with respect to 100 parts by weight of the base resin. Afterwards, a drying step was performed at 80 °C for 25 minutes.

**Example 2**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.3 parts by weight of a mixture A in an aqueous solution was mixed with the surface-treated super absorbent polymer with respect to 100 parts by weight of the base resin. Afterwards, a drying step was performed at 80 °C for 25 minutes.

**Example 3**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.5 parts by weight of a mixture A in an aqueous solution was mixed with the surface-treated super absorbent polymer with respect to 100 parts by weight of the base resin. Afterwards, a drying step was performed at 80 °C for 25 minutes.

**Example 4**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.1 parts by weight of a mixture B in an aqueous solution was mixed with the surface-treated super absorbent polymer with respect to 100 parts by weight of the base resin. Afterwards, a drying step was performed at 80 °C for 25 minutes.

**Example 5**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.3 parts by weight of a mixture B in an aqueous solution was mixed with the surface-treated super absorbent polymer with respect to 100 parts by weight of the base resin. Afterwards, a drying step was performed at 80 °C for 25 minutes.

**Example 6**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.5 parts by weight of a mixture B in an aqueous solution was mixed with the surface-treated super absorbent polymer with respect to 100 parts by weight of the base resin. Afterwards, a drying step was performed at 80 °C for 25 minutes.

**Example 7**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)). Further, 100 parts by weight of the base resin prepared above was mixed with 0.3 parts by weight of a mixture A in an aqueous solution. Then, a surface cross-linking reaction was performed at 140 °C for 30 minutes. After completion of the surface treatment, a super absorbent polymer with a particle diameter of 150 to 850 $\mu$m was obtained by classification using a sieve.

**Example 8**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 1.0 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.3 parts by weight of a mixture A in an aqueous solution was mixed with the surface-treated super absorbent polymer with respect to 100 parts by weight of the base resin. Afterwards, a drying step was performed at 80 °C for 25 minutes.

## Example 9

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

An EDTA aqueous solution (at an EDTA concentration of 40%) and a mixture A were mixed with the surface-treated super absorbent polymer. At this time, 0.5 parts by weight of EDTA was mixed with respect to 100 parts by weight of the base resin. In addition, 0.3 parts by weight of a mixture A in an aqueous solution was mixed with respect to 100 parts by weight of the base resin. Afterwards, a drying step was performed at 80 °C for 25 minutes.

## Example 10

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

An EDTA aqueous solution (at an EDTA concentration of 40%) and a mixture A were mixed with the surface-treated super absorbent polymer. At this time, 1.0 parts by weight of EDTA was mixed with respect to 100 parts by weight of the base resin. In addition, 0.3 parts by weight of a mixture A in an aqueous solution was mixed with respect to 100 parts by weight of the base resin. Afterwards, a drying step was performed at 80 °C for 25 minutes.

## Example 11

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. A GLDA aqueous solution (at a GLDA concentration of 40%) was sprayed onto the cut hydrogel polymer and mixed by chopping (hole size: 16 mm). At this time, 0.5 parts by weight of GLDA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.3 parts by weight of a mixture A in an aqueous solution was mixed with the surface-treated super absorbent polymer with respect to 100 parts by weight of the base resin. Afterwards, a drying step was performed at 80 °C for 25 minutes.

## Example 12

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. A GLDA aqueous solution (at a GLDA concentration of 40%) was sprayed onto the cut hydrogel polymer and mixed by chopping (hole size: 16 mm). At this time, 0.5 parts by weight of GLDA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

0.3 parts by weight of a mixture A in an aqueous solution was mixed with the surface-treated super absorbent polymer with respect to 100 parts by weight of the base resin. Afterwards, a drying step was performed at 80 °C for 25 minutes.

## Example 13

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

A mixture A and an iodine-based aqueous solution of $I_2$ and CuI (each at a concentration of 1%) were mixed with the surface-treated super absorbent polymer. At this time, 0.3 parts by weight of the mixture A in an aqueous solution was mixed with respect to 100 parts by weight of the base resin, and 1.0 parts by weight of the iodine-based aqueous solution was mixed with respect to 100 parts by weight of the base resin. Afterwards, a drying step was performed at 80 °C for 25 minutes.

**Example 14**

100 g of acrylic acid, 0.37 g of N,N'-methylenebisacrylamide as a cross-linking agent, 0.15 g of sodium persulfate (SPS) as a thermal initiator, 0.008 g of benzoin ether as a UV initiator, 40 g of caustic soda (NaOH), and 127 g of water were mixed to prepare an aqueous monomer composition with a monomer concentration of 45.8 wt%. The monomer aqueous composition was added to the supply part of a polymerization reactor equipped with a continuously moving conveyor belt, and then irradiated with ultraviolet rays using a UV irradiation device (irradiation amount: 10 mW/cm$^2$) while maintaining the polymerization temperature at 80 °C for 2 minutes to perform UV polymerization, thereby preparing a hydrogel polymer.

The prepared hydrogel polymer was in the form of a sheet, and this sheet was cut into small pieces. An EDTA aqueous solution (at an EDTA concentration of 40%) was sprayed onto the cut hydrogel polymer and mixed by chopping (hole size: 16 mm). At this time, 0.5 parts by weight of EDTA was mixed with respect to 100 parts by weight of the base resin.

The prepared hydrogel polymer was transferred to a meat chopper and cut to 2 mm to 10 mm. At this time, the moisture content of the cut hydrogel polymer was 47 wt%. Susbsequently, the hydrogel polymer was dried in a hot air dryer at a temperature of 170 °C for 30 minutes, and the dried hydrogel polymer was pulverized with a pin mill. Susbsequently, a base resin was prepared by classifying the polymer with a particle diameter of 150 $\mu$m to 850 $\mu$m using a sieve.

Thereafter, 100 parts by weight of the base resin prepared above was evenly mixed with a surface cross-linking solution (2.5 parts by weight of water, 0.1 parts by weight of ethylene glycol diglycidyl ether (EX-810), 0.1 parts by weight of aluminum sulfate 18 hydrate (Al-S) and 0.1 parts by weight of silica (Aerosil A200)), and then a surface cross-linking reaction was performed at 140 °C for 30 minutes. After completion of the surface treatment, a super absorbent polymer with a particle diameter of 150 to 850 $\mu$m was obtained by classification using a sieve.

A mixture A and citric acid were mixed with the surface-treated super absorbent polymer. At this time, 0.3 parts by weight of the mixture A in an aqueous solution was mixed with respect to 100 parts by weight of the base resin, and 1.0 parts by weight of the citric acid in an aqueous solution (at a concentration of 1%) was mixed with respect to 100 parts by weight of the base resin. Afterwards, a drying step was performed at 80 °C for 25 minutes.

[Table 1]

| Category | Chelating agent | Chelating agent usage (with respect to 100 parts by weight of base resin) | Mixture (with respect to 100 parts by weight of base resin) | Persimmon extract solid content (with respect to 100 parts by weight of base resin) | Glycine (with respect to 100 parts by weight of base resin) | Methionine (with respect to 100 parts by weight of base resin) | Other additive | Other additive usage (with respect to 100 parts by weight of base resin) |
|---|---|---|---|---|---|---|---|---|
| Ex. 1 | EDTA | 0.5 | Mixture A (0.1) | 0.005 | 0.005 | - | - | - |

(continued)

| Categor y | Chelatin g agent | Chelatin g agent usage (with respect to 100 parts by weight of base resin) | Mixtur e (with respe ct to 100 parts by weigh t of base resin) | Persimm on extract solid content (with respect to 100 parts by weight of base resin) | Glycin e (with respe ct to 100 parts by weight of base resin) | Methioni ne (with respect to 100 parts by weight of base resin) | Other additive | Other additiv e usage (with respec t to 100 parts by weight of base resin) |
|---|---|---|---|---|---|---|---|---|
| Ex. 2 | EDTA | 0.5 | Mixtur e A (0.3) | 0.015 | 0.015 | - | - | - |
| Ex. 3 | EDTA | 0.5 | Mixtur e A (0.5) | 0.025 | 0.025 | - | - | - |
| Ex. 4 | EDTA | 0.5 | Mixtur e B (0.1) | 0.005 | 0.007 | - | - | - |
| Ex. 5 | EDTA | 0.5 | Mixtur e B (0.3) | 0.015 | 0.021 | - | - | - |
| Ex. 6 | EDTA | 0.5 | Mixtur e B (0.5) | 0.025 | 0.035 | - | - | - |
| Ex. 7 | EDTA | 0.5 | Mixtur e A (0.3) | 0.015 | 0.015 | - | - | - |
| Ex. 8 | EDTA | 1.0 | Mixtur e A (0.3) | 0.015 | 0.015 | - | - | - |
| Ex. 9 | EDTA | 0.5 | Mixtur e A (0.3) | 0.015 | 0.015 | - | - | - |
| Ex. 10 | EDTA | 1.0 | Mixtur e A (0.3) | 0.015 | 0.015 | - | - | - |
| Ex. 11 | GLDA | 0.5 | Mixtur e A (0.3) | 0.015 | 0.015 | - | - | - |
| Ex. 12 | GLDA | 0.5 | Mixtur e A (0.3) | 0.015 | 0.015 | - | - | - |
| Ex. 13 | EDTA | 0.5 | Mixtur e A (0.3) | 0.015 | 0.015 | - | Iodine compou nd | 1.0 |
| Ex. 14 | EDTA | 0.5 | Mixtur e A (0.3) | 0.015 | 0.015 | - | Citric acid | 1.0 |
| Comp. Ex. 1 | - | - | - | - | - | - | - | - |
| Comp. Ex. 2 | EDTA | 0.5 | - | - | - | - | - | - |
| Comp. Ex. 3 | - | - | Mixtur e A (0.3) | 0.015 | 0.015 | - | - | - |
| Comp. Ex. 4 | EDTA | 0.5 | Mixtur e C (0.3) | 0.015 | - | 0.015 | - | - |
| Comp. Ex. 5 | EDTA | 0.5 | - | - | 0.015 | - | - | - |
| Comp. Ex. 6 | - | - | - | 0.015 | - | - | - | - |

(continued)

| Categor y | Chelatin g agent | Chelatin g agent usage (with respect to 100 parts by weight of base resin) | Mixtur e (with respe ct to 100 parts by weigh t of base resin) | Persimm on extract solid content (with respect to 100 parts by weight of base resin) | Glycin e (with respe ct to 100 parts by weight of base resin) | Methioni ne (with respect to 100 parts by weight of base resin) | Other additive | Other additiv e usage (with respec t to 100 parts by weight of base resin) |
|---|---|---|---|---|---|---|---|---|
| Comp. Ex. 7 | - | - | - | - | 0.015 | - | - | - |

## Experiment Examples

Physical properties of the super absorbent polymer compositions prepared in Examples and Comparative Examples were measured by the following method.

## 1) Bacterial growth inhibition rate

50 ml of synthetic urine inoculated with 3000 CFU/ml E.Coli. was added to 2 g of the super absorbent polymer of Comparative Example 1, and then incubated in an incubator at 35 °C for 12 hours. The incubated sample was washed with 150 mL of saline (0.9 wt% sodium chloride aqueous solution), and incubated on a Nutrient broth agar (BD DIFCO.) plate to measure CFU (Colony Forming Unit; CFU/ml) as the physical properties of the control group.

50 ml of synthetic urine inoculated with 3000 CFU/ml E.Coli. was added to 2 g of the super absorbent polymer prepared in one of Examples and Comparative Examples, and then incubated in an incubator at 35 °C for 12 hours. 150 mL of saline (0.9 wt% sodium chloride aqueous solution) was added to the incubated sample, and shaken for 1 minute to mix evenly. The resulting diluted solution was spread on a Nutrient broth agar (BD DIFCO.) plate, and incubated in an incubator at 30 °C for 24 hours to measure CFU (Colony Forming Unit; CFU/ml).

The bacterial growth inhibition rate defined by Equation 1 below was calculated using these measurement results, and antibacterial activity of each super absorbent polymer according to Examples and Comparative Examples was evaluated.

Bacterial growth inhibition rate = [1- {CFU(12hrs)/ CFU control(12hrs)}]*100 (%)                    [Equation 1]

In Equation 1,

CFU(12 hrs) represents the number of individuals of proliferated bacteria per unit volume of synthetic urine (CFU/ml), which was obtained by adding the super absorbent polymer to the synthetic urine inoculated with bacteria of E.Coli, and then incubating at 35 °C for 12 hours, and

CFU control(12 hrs) represents the number of individuals of proliferated bacteria per unit volume of synthetic urine (CFU/ml), which was obtained by incubating synthetic urine inoculated with the above bacteria on the super absorbent polymer of Comparative Example 1 under the same conditions, representing the number of individuals of bacteria per unit volume of synthetic urine (CFU/ml) measured for the control group.

## 2) Deodorizing ability

The deodorizing ability was measured using the adsorption chamber test method. 3-methylbutanal was selected as an aldehyde-based odorous substance, and dimethyltrisulfide (DMTS) was selected as a sulfur compound-based odorous substance.

- Adsorption chamber measurement method: 1g of super absorbent polymer was added to a 500mL glass bottle, and then 25mL of odorous substances were injected. Afterwards, aging was performed for 3 hours in a constant temperature chamber and then collection was performed for 20 minutes. At this time, the temperature of the constant temperature chamber was 35 °C and the $N_2$ flow rate was 250mL/min. The odor that was pushed out was then adsorbed to the connected adsorption chamber, and was collected after repeating the same operation twice for the same sample. The collection results were analyzed by GC to confirm the results.

-

Deodorizing ability (%) = (Odor amount of reference sample (super absorbent polymer of Comparative Example 1) measured by GC - Odor amount of sample measured by GC) / Odor amount of reference sample (super absorbent polymer of Comparative Example 1) measured by GC X 100(%)

### 3) Centrifuge retention capacity (CRC)

The centrifuge retention capacity by absorption ratio under a non-loading condition of each super absorbent polymer was measured in accordance with EDANA (European Disposables and Nonwovens Association, EDANA) WSP 241.3.

Specifically, polymers classified into a particle diameter of 300 to 600 $\mu$m were obtained from the polymers obtained in Examples and Comparative Examples. After inserting $W_0$ (g, about 0.2 g) of the polymer uniformly in a nonwoven fabric envelope and sealing the same, it was soaked in physiological saline (0.9 wt% sodium chloride aqueous solution) at room temperature. After 30 minutes, the envelope was centrifuged at 250G for 3 minutes to drain, and the weight $W_2$ (g) of the envelope was measured. Further, after conduction the same operation without using the polymer, the weight $W_1$ (g) of the envelope was measured.

Then, CRC (g/g) was calculated using the obtained weight values according to the following Calculation Equation 1.

[Calculation Equation 1]

$$CRC\ (g/g) = \{[W_2(g) - W_1(g)]/W_0(g)\} - 1$$

### 4) Absorbency under pressure (AUP)

The absorbency under pressure at 0.7 psi of the super absorbent polymer prepared in Examples and Comparative Examples was measured in accordance with EDANA WSP 242.3.

First, the classified polymers used in the above CRC measurement were used in the AUP measurement.

Specifically, a 400 mesh stainless steel screen was installed in a cylindrical bottom of a plastic having an inner diameter of 25 mm. $W_0$ (g) of the super absorbent polymer was uniformly scattered on the screen at room temperature and a humidity of 50%. Thereafter, a piston which can uniformly provide a load of 0.7 psi was placed thereon. Herein, the outer diameter of the piston was slightly smaller than 25 mm, there was no gap with the inner wall of the cylinder, and jig-jog of the cylinder was not interrupted. At this time, the weight $W_3$ (g) of the device was measured.

Subsequently, a glass filter having a diameter of 90 mm and a thickness of 5 mm was placed in a petri dish having a diameter of 150 mm, and saline (0.9 wt% sodium chloride) was poured in the dish. At this time, the saline was poured until the surface level of the saline became equal to the upper surface of the glass filter. One sheet of filter paper with a diameter of 90 mm was placed thereon. After the measuring device was placed on the filter paper, the liquid was absorbed for 1 hour under a load. After 1 hour, the measuring device was lifted, and the weight $W_4$ (g) was measured. Then, absorbency under pressure (g/g) was calculated by using the obtained mass values according to the following Calculation Equation 2.

[Calculation Equation 2]

$$AUP(g/g) = [W_4(g) - W_3(g)]/W_0(g)$$

The results of the experiment are shown in Table 2.

[Table 2]

| | Bacterial growth inhibition rate (%) | Deodorizing ability (%) | | Absorbency | |
| | | aldehyde-based | sulfur compound-based | CRC (g/g) | AUP (g/g) |
|---|---|---|---|---|---|
| Ex. 1 | 98 | 62 | 75 | 37.1 | 17.3 |
| Ex. 2 | 98 | 73 | 84 | 36.8 | 16.7 |
| Ex. 3 | 98 | 81 | 91 | 36.2 | 15.8 |
| Ex. 4 | 97 | 70 | 78 | 37.0 | 17.1 |

(continued)

| | Bacterial growth inhibition rate (%) | Deodorizing ability (%) | | Absorbency | |
| | | aldehyde-based | sulfur compound-based | CRC (g/g) | AUP (g/g) |
| --- | --- | --- | --- | --- | --- |
| Ex. 5 | 97 | 79 | 86 | 36.5 | 16.5 |
| Ex. 6 | 97 | 90 | 93 | 36.1 | 15.5 |
| Ex. 7 | 98 | 65 | 75 | 37.0 | 16.9 |
| Ex. 8 | 99.9 | 74 | 86 | 36.3 | 15.8 |
| Ex. 9 | 99.9 | 75 | 85 | 36.8 | 16.2 |
| Ex. 10 | 99.9 | 77 | 88 | 36.4 | 15.7 |
| Ex. 11 | 91 | 70 | 81 | 36.7 | 15.4 |
| Ex. 12 | 92 | 67 | 79 | 36.0 | 16.1 |
| Ex. 13 | 99.9 | 85 | 90 | 36.5 | 16.0 |
| Ex. 14 | 98 | 84 | 88 | 36.2 | 15.9 |
| Comp. Ex. 1 | 0 | 0 | 0 | 37.5 | 17.8 |
| Comp. Ex. 2 | 96 | 7 | 5 | 37.3 | 17.7 |
| Comp. Ex. 3 | 0 | 63 | 71 | 37.2 | 17.4 |
| Comp. Ex. 4 | 97 | 56 | 65 | 36.5 | 16.0 |
| Comp. Ex. 5 | 97 | 35 | 30 | 37.1 | 17.0 |
| Comp. Ex. 6 | 0 | 55 | 64 | 37.3 | 17.5 |
| Comp. Ex. 7 | 0 | 15 | 30 | 37.4 | 17.5 |

According to the results in Table 2, Examples showed excellent bacterial growth inhibition rate and deodorizing ability while maintaining the absorbency at a similar level compared to Comparative Example 1 containing no additives.

In addition, in the case of Comparative Example 2 where only a chelating agent was used without a deodorizing agent, the bacterial growth inhibition rate was at a similar level, but the deodorizing ability was very poor. Comparative Examples 3, 6, and 7, which used only a deodorizing agent without a chelating agent, did not inhibit bacteria at all, and their deodorizing ability tended to be lower than that of Examples.

Comparative Example 4, where methionine was used instead of glycine, did not show an excellent deodorizing effect on aldehyde-based and sulfur compound-based substances at the same time unlike the Examples.

In the case of Comparative Example 5 including a chelating agent and glycine without persimmon extract, it was confirmed that the deodorizing ability for both aldehyde-based and sulfur compound-based substances was inferior to that of Examples.

**Claims**

1. A super absorbent polymer composition comprising

   a super absorbent polymer comprising a base resin containing a cross-linked polymer in which an acrylic acid-based monomer having at least partially neutralized acidic groups and an internal cross-linking agent are cross-linking polymerized, and a surface cross-linked layer formed by further cross-linking the cross-linked polymer using a surface cross-linking agent on a surface of the base resin;
   persimmon extract;
   glycine; and
   a chelating agent.

2. The super absorbent polymer composition of Claim 1,
   wherein the persimmon extract is included in an amount of 0.005 to 0.050 parts by weight based on the solid content with respect to 100 parts by weight of the base resin.

3. The super absorbent polymer composition of Claim 1,
wherein the glycine is included in an amount of 0.005 to 0.050 parts by weight with respect to 100 parts by weight of the base resin.

4. The super absorbent polymer composition of Claim 1,
wherein the glycine is included in an amount of 100 to 200 parts by weight with respect to 100 parts by weight of the persimmon extract based on the solid content.

5. The super absorbent polymer composition of Claim 1,
wherein the chelating agent is included in an amount of 0.1 to 2.0 parts by weight with respect to 100 parts by weight of the base resin.

6. The super absorbent polymer composition of Claim 1,
wherein the super absorbent polymer composition further comprises one or more additives selected from the group consisting of an organic acid, an iodine compound, glycerin, and zinc chloride.

7. The super absorbent polymer composition of Claim 1,
wherein the chelating agent comprises an amino acetate-based chelating agent.

8. The super absorbent polymer composition of Claim 7,
wherein the amino acetate-based chelating agent comprises at least one selected from the group consisting of ethylenediaminetetraacetic acid (EDTA), L-glutamic acid diacetic acid (GLDA), methyl glycine diacetic acid (MGDA), hydroxyethylethylenediaminetriacetic acid (HEDTA), ethanoldiglycine (EDG), diethylenetriaminepentaacetic acid (DTPA), and salts thereof.

9. The super absorbent polymer composition of Claim 1,
wherein the persimmon extract, glycine, and chelating agent are each independently included inside the base resin or inside the surface cross-linked layer.

10. A preparation method of a super absorbent polymer composition comprising the steps of:

forming a hydrogel polymer by performing cross-linking polymerization of an acrylic acid-based monomer having at least partially neutralized acidic groups in presence of an internal cross-linking agent and a polymerization initiator (step 1);
preparing a base resin containing a cross-linked polymer obtained by drying and pulverizing the hydrogel polymer (step 2);
preparing a mixture by mixing a surface cross-linking solution with the base resin (step 3); and
preparing a super absorbent polymer with a surface cross-linked layer formed on a surface of the base resin by heat-treating the mixture (step 4);
wherein a mixture containing persimmon extract and glycine is mixed with the surface cross-linking solution of step 3, or mixed with the super absorbent polymer on which the surface cross-linked layer is formed of step 4, and mixing a chelating agent with the hydrogel polymer of step 1, mixed with the surface cross-linking solution of step 3, or mixed with the super absorbent polymer on which the surface cross-linked layer is formed of step 4.

11. The preparation method of a super absorbent polymer composition of Claim 10,
wherein the persimmon extract is mixed in an amount of 0.005 to 0.050 parts by weight based on the solid content with respect to 100 parts by weight of the base resin.

12. The preparation method of a super absorbent polymer composition of Claim 10,
wherein the glycine is mixed in an amount of 0.005 to 0.050 parts by weight with respect to 100 parts by weight of the base resin.

13. The preparation method of a super absorbent polymer composition of Claim 10,
wherein the glycine is included in an amount of 100 to 200 parts by weight with respect to 100 parts by weight of the persimmon extract based on solid content.

14. The preparation method of a super absorbent polymer composition of Claim 10,
wherein the chelating agent is mixed in an amount of 0.1 to 2.0 parts by weight with respect to 100 parts by weight of the base resin.

15. The preparation method of a super absorbent polymer composition of Claim 10, further mixing one or more additives selected from the group consisting of an organic acid, an iodine compound, glycerin, and zinc chloride with the super absorbent polymer on which a surface cross-linked layer is formed after step 4.

16. The preparation method of a super absorbent polymer composition of Claim 10, wherein the chelating agent comprises an amino acetate-based chelating agent.

17. The preparation method of a super absorbent polymer composition of Claim 16 wherein the amino acetate-based chelating agent comprises at least one selected from the group consisting of ethylenediaminetetraacetic acid (EDTA), L-glutamic acid diacetic acid (GLDA), methyl glycine diacetic acid (MGDA), hydroxyethylethylenediaminetriacetic acid (HEDTA), ethanoldiglycine (EDG), diethylenetriaminepentaacetic acid (DTPA), and salts thereof.

## INTERNATIONAL SEARCH REPORT

| | International application No. |
|---|---|
| | **PCT/KR2023/019329** |

### A. CLASSIFICATION OF SUBJECT MATTER

**C08K 11/00**(2006.01)i; **C08K 5/17**(2006.01)i; **C08K 5/09**(2006.01)i; **C08K 5/053**(2006.01)i; **C08K 3/16**(2006.01)i; **C08F 20/06**(2006.01)i; **C08J 3/24**(2006.01)i

According to International Patent Classification (IPC) or to both national classification and IPC

### B. FIELDS SEARCHED

Minimum documentation searched (classification system followed by classification symbols)

C08K 11/00(2006.01); A23K 10/20(2016.01); A23K 10/30(2016.01); A61F 13/15(2006.01); A61L 15/60(2006.01); A61L 9/01(2006.01); C08J 3/24(2006.01); C08K 3/00(2006.01); C08K 5/00(2006.01); D06M 13/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 고흡수성 수지(super absorption polymer), 아크릴산(acrylic acid), 표면 가교 (surface crosslinking), 감나무 추출물(extract of persimmon tree), 글리신(glycine), 킬레이트제(chelating agent)

### C. DOCUMENTS CONSIDERED TO BE RELEVANT

| Category* | Citation of document, with indication, where appropriate, of the relevant passages | Relevant to claim No. |
|---|---|---|
| Y | KR 10-2019-0125027 A (LG CHEM, LTD.) 06 November 2019 (2019-11-06)<br>See paragraphs [0003], [0037], [0061], [0073] and [0096]-[0102]; and claims 1, 5-6 and 13. | 1-17 |
| Y | JP 2002-146673 A (DAINIPPON JOCHUGIKU CO., LTD.) 22 May 2002 (2002-05-22)<br>See paragraph [0009]; and claim 1. | 1-17 |
| A | KR 10-2019-0035313 A (LG CHEM, LTD.) 03 April 2019 (2019-04-03)<br>See entire document. | 1-17 |
| A | KR 10-2007-0104663 A (NIPPON SHOKUBAI CO., LTD.) 26 October 2007 (2007-10-26)<br>See entire document. | 1-17 |
| A | KR 10-2020-0094979 A (NATUREFRENDLY CO., LTD. et al.) 10 August 2020 (2020-08-10)<br>See entire document. | 1-17 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **12 March 2024** | **12 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

<table>
<tr><td colspan="2" align="center">INTERNATIONAL SEARCH REPORT<br>Information on patent family members</td><td colspan="3">International application No.<br><b>PCT/KR2023/019329</b></td></tr>
</table>

| Patent document cited in search report | | | Publication date (day/month/year) | Patent family member(s) | | | Publication date (day/month/year) |
|---|---|---|---|---|---|---|---|
| KR | 10-2019-0125027 | A | 06 November 2019 | CN | 111315808 | A | 19 June 2020 |
| | | | | CN | 111315808 | B | 18 October 2022 |
| | | | | EP | 3677628 | A1 | 08 July 2020 |
| | | | | EP | 3677628 | A4 | 23 December 2020 |
| | | | | JP | 2020-528945 | A | 01 October 2020 |
| | | | | JP | 6937888 | B2 | 22 September 2021 |
| | | | | KR | 10-2452566 | B1 | 06 October 2022 |
| | | | | US | 11718742 | B2 | 08 August 2023 |
| | | | | US | 2020-0270441 | A1 | 27 August 2020 |
| | | | | WO | 2019-208905 | A1 | 31 October 2019 |
| JP | 2002-146673 | A | 22 May 2002 | JP | 4702992 | B2 | 15 June 2011 |
| KR | 10-2019-0035313 | A | 03 April 2019 | KR | 10-2487978 | B1 | 11 January 2023 |
| KR | 10-2007-0104663 | A | 26 October 2007 | AU | 2006-216015 | A1 | 24 August 2006 |
| | | | | BR | PI0607725 | A2 | 06 October 2009 |
| | | | | BR | PI0607725 | B1 | 04 July 2017 |
| | | | | CN | 101120038 | A | 06 February 2008 |
| | | | | CN | 104086938 | A | 08 October 2014 |
| | | | | CN | 104086938 | B | 19 September 2017 |
| | | | | CN | 1847289 | A | 18 October 2006 |
| | | | | CN | 1847289 | B | 14 December 2011 |
| | | | | EP | 1690887 | A1 | 16 August 2006 |
| | | | | EP | 1690887 | B1 | 21 May 2008 |
| | | | | EP | 1848758 | A1 | 31 October 2007 |
| | | | | EP | 1848758 | A4 | 30 November 2011 |
| | | | | EP | 1848758 | B1 | 07 November 2012 |
| | | | | EP | 1848758 | B2 | 17 February 2021 |
| | | | | JP | 2006-297373 | A | 02 November 2006 |
| | | | | JP | 2006-299234 | A | 02 November 2006 |
| | | | | JP | 2012-086221 | A | 10 May 2012 |
| | | | | JP | 2014-039927 | A | 06 March 2014 |
| | | | | JP | 2016-033224 | A | 10 March 2016 |
| | | | | JP | 4965865 | B2 | 04 July 2012 |
| | | | | JP | 5046522 | B2 | 10 October 2012 |
| | | | | JP | 5625003 | B2 | 12 November 2014 |
| | | | | JP | 5903086 | B2 | 13 April 2016 |
| | | | | JP | 6025953 | B2 | 16 November 2016 |
| | | | | MX | 2007009284 | A | 29 January 2008 |
| | | | | RU | 2007-134385 | A | 27 March 2009 |
| | | | | RU | 2364611 | C2 | 20 August 2009 |
| | | | | SG | 159540 | A1 | 30 March 2010 |
| | | | | TW | 200706566 | A | 16 February 2007 |
| | | | | TW | I406883 | B | 01 September 2013 |
| | | | | US | 2006-0183828 | A1 | 17 August 2006 |
| | | | | US | 2010-0062252 | A1 | 11 March 2010 |
| | | | | US | 8182916 | B2 | 22 May 2012 |
| | | | | WO | 2006-088115 | A1 | 24 August 2006 |
| KR | 10-2020-0094979 | A | 10 August 2020 | KR | 10-2211027 | B1 | 08 February 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220182257

- KR 1020230164000

**Non-patent literature cited in the description**

- **REINHOLD SCHWALM**. UV Coatings: Basics, Recent Developments and New Application. Elsevier, 2007, 115

- **ODIAN**. Principle of Polymerization. Wiley, 1981, 203